# FASCICULE DE BREVET EUROPEEN

(11) **EP 0 974 335 B1**
(45) Date de publication et mention de la délivrance du brevet: **10.09.2003**
(21) Numéro de dépôt: 99401699.6
(22) Date de dépôt: 07.07.1999
(51) Int. Cl.: A61K 7/06, A61K 7/48, A61K 7/50

(54) **Compositions cosmétiques détergentes et utilisation**
Kosmetische Tensid Zusammensetzung sowie ihre Verwendung
Cosmetic detergent compositions and their use

(30) Priorité: 23.07.1998 FR 9809414
(43) Date de publication de la demande: 26.01.2000
(73) Titulaire: L'OREAL, 75008 Paris (FR)
(72) Inventeur: Restle, Serge, 95390 St. Prix (FR); Dubief, Claude, 78150 Le Chesnay (FR)
(74) Mandataire: Le Blainvaux Bellegarde, Françoise

(56) Documents cités:
- EP-A- 0 521 748
- EP-A- 0 870 491
- WO-A-93/08787
- WO-A-94/06403
- WO-A-95/01152
- WO-A-96/32919
- FR-A- 2 761 598

## Description

La présente invention concerne de nouvelles compositions cosmétiques à propriétés améliorées destinées simultanément au nettoyage et au conditionnement des matières kératiniques telles que les cheveux, et comprenant, dans un support aqueux cosmétiquement acceptable, une base lavante constituée de tensioactifs anioniques et amphotères dans laquelle est également présent une silicone aminée ayant un indice d'amine supérieur ou égal à 0,4 meq./g. L'invention concerne aussi l'utilisation desdites compositions dans l'application cosmétique susmentionnée.

Pour le nettoyage et/ou le lavage des matières kératiniques telles que les cheveux, l'utilisation de compositions détergentes (telles que les shampooings) à base essentiellement d'agents tensioactifs classiques de type notamment anionique, non ionique et/ou amphotère, mais plus particulièrement de type anionique, est courante. Ces compositions sont appliquées sur cheveux mouillés et la mousse générée par massage ou friction avec les mains permet, après rinçage à l'eau, l'élimination des diverses salissures initialement présentes sur les cheveux ou la peau.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur la fibre capillaire des dommages plus ou moins marqués liés en particulier à l'élimination progressive des lipides ou protéines contenues dans ou à la surface de cette dernière.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

Dans ce but, on a déjà proposé d'utiliser des silicones et plus particulièrement des silicones insolubles. Les composés insolubles et plus particulièrement les silicones présentent l'inconvénient d'être difficiles à maintenir en dispersion régulière dans le milieu.

Pour maintenir en suspension les silicones, on a déjà proposé l'utilisation des dérivés d'esters ou d'éthers à longue chaîne (agents nacrants) ou des polysaccharides tels que la gomme de xanthane (gélifiants). Cependant, les agents nacrants présentent des problèmes de cristallisation qui entraînent parfois une évolution (augmentation) de la viscosité des compositions au cours du temps ; les agents gélifiant présentent également des inconvénients, à savoir d'une part que la mousse des compositions détergentes contenant des polysaccharides se développe difficilement (mauvais démarrage de mousse) et que, d'autre part, les compositions n'ont pas une texture lisse et s'écoulent par paquets, ce qui est peu apprécié des utilisateurs. De plus, ces divers agents de suspension ne permettent pas d'obtenir des compositions transparentes ou limpides.

La présente invention a pour but de proposer des compositions ne présentant pas les inconvénients des compositions citées ci-dessus.

On connaît dans les documents WO95/01152, EP0521748, WO93/08787, WO96/32919 et WO94/06403 des compositions de shampooing comprenant un tensioactif anionique, éventuellement un tensioactif amphotère et une silicone telle qu'un polydiméthylsiloxane. La demande EP0870491 publiée le 14/10/1998 décrit des compositions comprenant un tensioactif anionique, un tensioactif amphotère et une silicone aminée, la DC939 dont l'indice d'amine est inférieur à 0,1 meq./g.

Les silicones doivent également être véhiculées sur les matières kératiniques traitées en vue de leur conférer, suivant l'application, des propriétés de douceur, de brillance et de démêlage sans induire de caractère gras.

Ainsi, à la suite d'importantes recherches menées sur la question, il a maintenant été trouvé par la Demanderesse, qu'en utilisant une base lavante particulière et au moins une silicone aminée ayant un indice d'amine supérieur ou égal à 0,4 meq./g, il est possible d'obtenir des compositions détergentes stables et transparentes présentant d'excellentes propriétés cosmétiques, en particulier la facilité de coiffage, le démêlage et le volume des cheveux traités et ayant de bonnes propriétés d'usage tel qu'un bon pouvoir lavant intrinsèque et un bon pouvoir moussant.

La mise en oeuvre industrielle est extrêmement facile et les propriétés cosmétiques des shampooings sont excellentes.

Les compositions conformes à l'invention confèrent aux cheveux, après rinçage, un remarquable effet traitant qui se manifeste notamment par une facilité de démêlage, ainsi qu'un apport de volume, de légèreté, de lissage, de douceur et de souplesse sans aucune sensation de gras.

Ainsi, la présente invention a pour objet de nouvelles compositions cosmétiques détergentes et conditionnantes caractérisée par le fait qu'elles comprennent, dans un milieu aqueux cosmétiquement acceptable, (A) une base comprenant au moins un tensioactif anionique et au moins un tensioactif amphotère, (B) un système conditionneur comprenant au moins une silicone aminée dont l'indice d'amine est supérieur ou égal à 0,4 meq./g, choisie parmi :
(a) les polymères cationiques siliconés répondant à la formule :

   R'ₐG₃₋ₐ-Si(OSiG₂)ₙ-(OSiG_{b}R'_{2-b})ₘ-O-SiG₃₋ₐ-R'ₐ (I)

   dans laquelle :
   G est un atome d'hydrogène, ou un groupement phényle, OH, ou alkyle en C₁-C₈, par exemple méthyle,
   a désigne le nombre 0 ou un nombre entier de 1 à 3, en particulier 0,
   b désigne 0 ou 1, et en particulier 1,
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
   R' est un radical monovalent de formule -C_{q}H_{2q}L dans laquelle q est un nombre de 2 à 8 et L est un groupement aminé éventuellement quaternisé choisi parmi les groupements :
      -N(R")-CH₂-CH₂-N(R")₂
      -N(R")₂
      -N^{⊕}(R")₃A⁻
      -N^{⊕}H(R")₂A⁻
      -N^{⊕}H₂(R")A⁻
      -N(R")-CH₂-CH₂-N^{⊕}R"H₂A⁻,
      dans lesquels R", identiques ou différents, peut désigner hydrogène, phényle, benzyle, ou un radical hydrocarboné saturé monovalent, par exemple un radical alkyle ayant de 1 à 20 atomes de carbone, de préférence méthyle et A⁻ représente un anion organique ou minéral.
(b) les polymères cationiques siliconés répondant à la formule (IV) suivante : 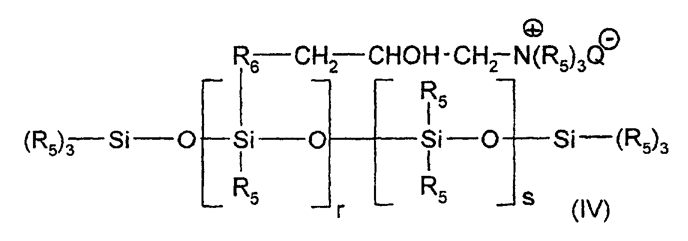
dans laquelle
R₅ représente un radical hydrocarboné monovalent ayant de 1 à 18 atomes de carbone, et en particulier un radical alkyle en C₁-C₁₈, ou alcényle en C₂-C₁₈, par exemple méthyle ;
R₆ représente un radical hydrocarboné divalent, notamment un radical alkylène en C₁-C₁₈ ou un radical alkylèneoxy divalent en C₁-C₁₈, par exemple en C₁-C₈ ;
Q⁻ est un anion organique ou minéral ;
r représente une valeur statistique moyenne de 2 à 20 et en particulier de 2 à 8 ;
s représente une valeur statistique moyenne de 20 à 200 et en particulier de 20 à 50,
le rapport en poids tensioactif amphotère / tensioactif anionique étant supérieur ou égal à 0,2.

L'invention a également pour objet l'utilisation en cosmétique des compositions ci-dessus pour le nettoyage et /ou le démaquillage et/ou le conditionnement des matières kératiniques telles que les cheveux et la peau.

### A- BASE LAVANTE :

La base lavante comprend un ou plusieurs tensioactifs anioniques et un ou plusieurs tensioactifs amphotères.

### (i) Tensioactif(s) anionique(s) :

Leur nature ne revêt pas, dans le cadre de la présente invention, de caractère véritablement critique.

Ainsi, à titre d'exemple de tensioactifs anioniques utilisables, seuls ou en mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 12 à 20 atomes de carbone, et le radical aryle désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.

On utilise de préférence un agent tensioactif anionique choisi parmi les alkyl(C₁₂-C₁₄) sulfates de sodium, de triéthanolamine ou d'ammonium, les alkyl (C₁₂-C₁₄)éthersulfates de sodium oxyéthylénés à 2,2 moles d'oxyde d'éthylène, le cocoyl iséthionate de sodium et l'alphaoléfine(C₁₄-C₁₆) sulfonate de sodium.

Parmi les tensioactifs anioniques, on préfère utiliser selon l'invention les sels d'alkylsulfates et d'alkyléthersulfates et leurs mélanges.

### (iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL®, tels que décrits dans les brevets US-2 528 378 et US-2 781 354 et de structures :

R₂-CONHCH₂CH₂-N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R_{2'}-CONHCH₂CH₂-N(B)(C) (3)

dans laquelle :
B représente -CH₂CH₂OX', C représente -(CH₂)_{z}-Y', avec z = 1 ou 2,
X' désigne le groupement -CH₂CH₂-COOH ou un atome d'hydrogène
Y' désigne -COOH ou le radical -CH₂-CHOH - SO₃H
R_{2'} désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL® C2M concentré par la société RHONE POULENC.

Selon la présente invention, on préfère plus particulièrement utiliser les agents tensio-actifs amphotères appartenant au groupe des bétaïnes tels que les alkylbétaïnes en particulier la cocoylbétaïne commercialisée sous la dénomination « DEHYTON AB 30 » en solution aqueuse à 30 % de MA par la société HENKEL ou les alkylamidobétaines telles que la TEGOBETAINE® F50 commercialisée par la société GLODSCHMIDT.

La quantité minimale de base lavante est celle juste suffisante pour conférer à la composition finale un pouvoir moussant et/ou détergent satisfaisant, et des quantités trop importantes de base lavante n'apportent pas vraiment d'avantages supplémentaires.

Ainsi, selon l'invention, la base lavante peut représenter de 4 % à 50 % en poids, de préférence de 6 % à 35 % en poids, et encore plus préférentiellement de 8 % à 25 % en poids, du poids total de la composition finale.

A titre indicatif, les compositions détergentes conformes à l'invention présentent généralement les compositions suivantes :
(i) tensio-actif(s) anionique(s) : de 3 à 30 % en poids, de préférence de 5 à 20 % en poids, par rapport au poids total de la composition détergente ;
(ii) tensio-actif(s) amphotère(s): de 1 à 20 % en poids, de préférence de 1,5 à 15 % en poids, par rapport au poids total de la composition.

Le rapport en poids tensioactif amphotère / tensioactif anionique est de préférence compris entre 0,2 et 10, plus particulièrement entre 0,25 et 5 et encore plus particulièrement entre 0,3 et 3.

### B- SILICONE AMINEE

Les compositions selon l'invention comprennent nécessairement une silicone aminée, insoluble dans l'eau, dont l'indice d'amine est supérieur ou égal à 0,4 meq./g, de préférence compris entre 0,5 et 5 meq./g, et plus particulièrement entre 0,5 et 3,5 meq./g.

L'indice d'amine est le nombre de milli-équivalents amine par gramme de composé. Cet indice est déterminé de façon tout à fait classique par des méthodes de titrage par indicateur coloré ou par titrage potentiométrique.

Par insoluble dans l'eau, on comprend qu'une solution à une concentration de 1% en poids dans l'eau n'est pas substantiellement transparente à l'oeil nu à 25°C.

Selon l'invention, on désigne par silicone aminée toute silicone comportant au moins une amine primaire, secondaire, tertiaire ou un groupement ammonium quaternaire. On peut ainsi citer :
(a) les polymères cationiques siliconés répondant à la formule :

   R'ₐG₃₋ₐ-Si(OSiG₂)ₙ-(OSiG_{b}R'_{2-b})ₘ-O-SiG₃₋ₐ-R'ₐ (I)

   dans laquelle :
   G est un atome d'hydrogène, ou un groupement phényle, OH, ou alkyle en C₁-C₈, par exemple méthyle,
   a désigne le nombre 0 ou un nombre entier de 1 à 3, en particulier 0,
   b désigne 0 ou 1, et en particulier 1,
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
   R' est un radical monovalent de formule -C_{q}H_{2q}L dans laquelle q est un nombre de 2 à 8 et L est un groupement aminé éventuellement quaternisé choisi parmi les groupements :
      -N(R")-CH₂-CH₂-N(R")₂
      -N(R")₂
      -N^{⊕}(R")₃A⁻
      -N^{⊕}H(R")₂A⁻
      -N^{⊕}H₂(R")A⁻
      -N(R")-CH₂-CH₂-N^{⊕}R"H₂A⁻,
      dans lesquels R", identiques ou différents, peut désigner hydrogène, phényle, benzyle, ou un radical hydrocarboné saturé monovalent, par exemple un radical alkyle ayant de 1 à 20 atomes de carbone, de préférence méthyle et A⁻ représente un anion organique ou minéral par exemple un ion halogénure tel que par exemple fluorure, chlorure, bromure ou iodure.

   Des produits correspondant à cette définition sont notamment :
   - le polymère dénommé "triméthylsilylamodiméthicone" (International Cosmetic Ingredient Dictionary anh Handbook Seventh Edition 1997), répondant à la formule : 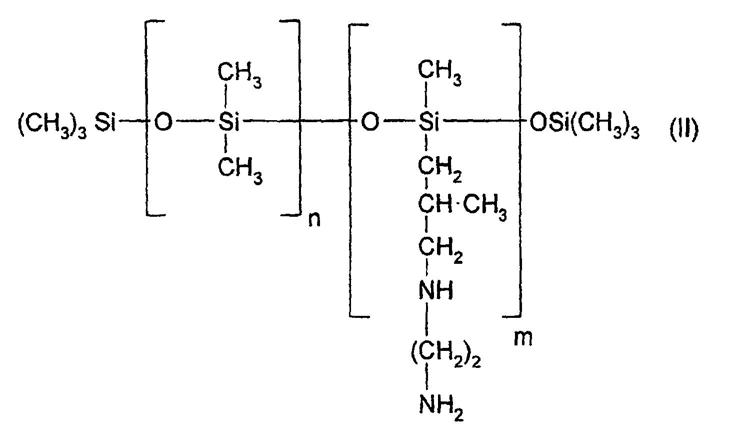 dans laquelle n et m ont les significations données ci-dessus (cf formule I).
   - le polymère dénommé "amodiméthicone" (International Cosmetic Ingrédient Dictionary anh Handbook Seventh Edition 1997) répondant à la formule : 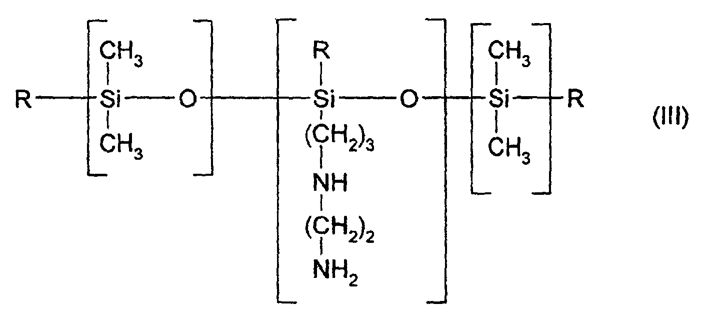 dans laquelle R désigne OH ou méthyle.

   De tels polymères sont décrits par exemple dans la demande de brevet EP-A-95238.
(b) les polymères cationiques siliconés répondant à la formule (IV) suivante : 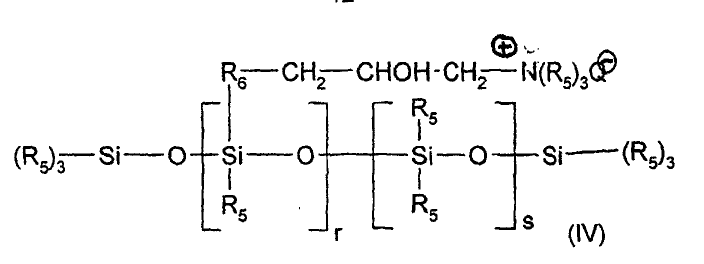
dans laquelle
R₅ représente un radical hydrocarboné monovalent ayant de 1 à 18 atomes de carbone, et en particulier un radical alkyle en C₁-C₁₈, ou alcényle en C₂-C₁₈, par exemple méthyle ;
R₆ représente un radical hydrocarboné divalent, noatmment un radical alkylène en C₁-C₁₈ ou un radical alkylèneoxy divalent en C₁-C₁₈, par exemple en C₁-C₈ ;
Q⁻ est un anion organique ou minéral par exemple un ion halogénure, notamment chlorure ;
r représente une valeur statistique moyenne de 2 à 20 et en particulier de 2 à 8 ;
s représente une valeur statistique moyenne de 20 à 200 et en particulier de 20 à 50.

De tels polymères sont décrits plus particulièrement dans le brevet US 4 185 087.

Des silicones aminées convenant particulièrement bien selon l'invention sont notamment les triméthylsilylamodiméthicones commercialisées par la société WACKER sous la dénomination FINISH WT 1650, par la société General Electric sous la dénomination SF 1708, les amodiméthicones commercialisées par la société WACKER sous les dénominations FINISH WT 1600 et L 650, par la société GENESE sous la dénomination SP4 Silicone Fluid.

La ou les silicones peuvent être utilisées dans les compositions conformes à l'invention dans des concentrations généralement comprises entre 0,05 et 15 %, et de préférence entre 0,2 et 10 % en poids par rapport au poids total de la composition et encore plus particulièrement de 0,5 à 5% en poids.

Le milieu aqueux cosmétiquement acceptable peut être constitué uniquement par de l'eau ou par un mélange d'eau et d'un solvant cosmétiquement acceptable tel qu'un alcool inférieur en C₁-C₁₂, comme l'éthanol, l'isopropanol, le tertiobutanol, le n-butanol, l'hexanol, le décanol ; les polyols tels que les alkylèneglycols comme le propylèneglycol, la glycérine et les polyalkylèneglycols ; les éthers de glycols.

Le ou les solvant peuvent être utilisés dans des concentrations généralement comprises entre 0,1 et 20% en poids et plus particulièrement entre 0,2 et 10% en poids.

Les compositions détergentes selon l'invention présentent un pH final généralement compris entre 3 et 8. De préférence, ce pH est compris entre 4 et 6,5. L'ajustement du pH à la valeur désirée peut se faire classiquement par ajout d'une base (organique ou minérale) dans la composition, par exemple de la soude, de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide minéral ou organique, de préférence l'acide citrique ou l'acide chlorhydrique.

Les compositions conformes à l'invention peuvent contenir en plus de l'association définie ci-dessus des agents régulateurs de viscosité tels que des électrolytes, ou des agents épaississants. On peut citer en particulier le chlorure de sodium, le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères acide acrylique / acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir jusqu'à 5 % d'agents nacrants ou opacifiants bien connus dans l'état de la technique tels que par exemple les alcools gras, les palmitates de sodium ou de magnésium, les stéarates et hydroxystéarates de sodium ou de magnésium, les alcool gras, les dérivés acylés à chaîne grasse tels que les monostéarates ou distéarates d'éthylène glycol ou de polyéthylèneglycol, les éthers à chaînes grasses tels que par exemple le distéaryléther ou le 1-(hexadécyloxy)-2-octadécanol.

Les compositions conformes à l'invention peuvent éventuellement contenir en outre d'autres agents ayant pour effet d'améliorer les propriétés cosmétiques de cheveux ou de la peau sans cependant altérer la stabilité des compositions. On peut citer à ce sujet les agents tensioactifs cationiques, les polymères anioniques ou non ioniques ou cationiques ou amphotères, les protéines, les hydrolysats de protéines, les céramides, les pseudocéramides, les acides gras à chaînes linéaires ou ramifiées en C₁₆-C₄₀ tels que l'acide méthyl-18 eicosanoïque, les hydroxyacides, les vitamines, le panthénol, les silicones différentes des silicones de l'invention, volatiles ou non volatiles, solubles et insolubles dans le milieu, les filtres UV, les agents hydratants, les agents antipelliculaires ou antiséborrhéiques, les agents anti-radicaux libres, et leurs mélanges.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière encore plus générale, au sens de la présente invention, l'expression « polymère cationique » désigne tout polymère contenant des groupements cationiques et/ou des groupements ionisables en groupements cationiques.

Parmi tous les polymères cationiques susceptibles d'être utilisés dans le cadre de la présente invention, on préfère mettre en oeuvre les dérivés d'éther de cellulose quaternaires tels que les produits commercialisés sous la dénomination « JR 400 » par la société UNION CARBIDE CORPORATION, les cyclopolymères, en particulier les homopolymères de sel de diallyldiméthylammonium et les copolymères de sel de diallyldiméthylammonium et d'acrylamide en particulier les chlorures, commercialisés sous les dénominations «MERQUAT 100», «MERQUAT 550» et «MERQUAT S» par la société MERCK, les polysaccharides cationiques et plus particulièrement les gommes de guar modifiées par du chlorure de 2,3-époxypropyl triméthylammonium commercialisées par exemple sous la dénomination « JAGUAR C13S » par la société MEYHALL, les homopolymères et les copolymères éventuellement réticulés de sel de (méth)acryloyloxyéthyltriméthylammonium, vendus par la société ALLIED COLLOIDS en solution à 50% dans de l'huile minérale sous les dénominations commerciales SALCARE SC92 (copolymère réticulé du chlorure de méthacryloyloxyéthyltriméthylammonium et de l'acrylamide) et SALCARE SC95 (homopolymère réticulé du chlorure de méthacryloyloxyéthyl triméthylammonium), les copolymères de vinylpyrrolidone et de sel deméthylvinyl imidazolium tels que les produits commercialisés par BASF sous les dénominations LUVIQUAT FC 370, LUVIQUAT FC 550, LUVIQUAT FC 905 et LUVIQUAT HM-552.

On peut également utiliser les polymères qui sont constitués de motifs récurrents répondant à la formule 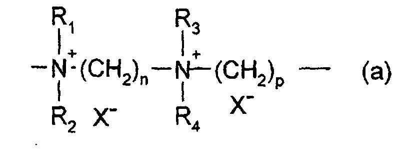 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.

Un composé de formule (a) particulièrement préféré est celui pour lequel R₁, R₂, R₃ et R₄, représentent un radical méthyle et n = 3, p = 6 et X = Cl, dénommé Hexadimethrine chloride selon la nomenclature INCI (CTFA).

Selon l'invention, le ou les polymères cationiques peuvent représenter de 0,001 % à 10 % en poids, de préférence de 0,005 % à 5 % en poids, et encore plus préférentiellement de 0,01 % à 3 % en poids, du poids total de la composition finale.

Les compositions selon l'invention peuvent contenir également des synergistes de mousses tels que des 1,2-alcanediols en C₁₀-C₁₈ ou des alcanolamides gras dérivés de mono ou de diéthanolamine.

Bien entendu, l'homme du métier veillera à choisir ce ou ces éventuels composés complémentaires et/ou leurs quantités de manière telle que les propriétés avantageuses attachées intrinsèquement à l'association conforme à l'invention ne soient pas, ou substantiellement pas, altérées par la ou les adjonctions envisagées.

Le pouvoir moussant des compositions selon l'invention, caractérisé par une hauteur de mousse, est généralement supérieur à 75 mm ; de préférence, supérieure à 100 mm mesurée selon la méthode ROSS-MILES (NF T 73-404 /ISO696) modifiée.
Les modifications de la méthode sont les suivantes :
La mesure se fait à la température de 22°C avec de l'eau osmosée. La concentration de la solution est de 2g/l. La hauteur de la chute est de 1m. La quantité de composition qui chute est de 200 ml. Ces 200 ml de composition tombe dans une éprouvette ayant un diamètre de 50 mm et contenant 50 ml de la composition à tester. La mesure est faite 5 minutes après l'arrêt de l'écoulement de la composition.

Ces compositions peuvent se présenter sous la forme de liquides plus ou moins épaissis, de crèmes ou de gels et elles conviennent principalement au lavage, au soin des matières kératiniques en particulier des cheveux et de la peau et encore plus particulièrement des cheveux.

Généralement, la silicone aminée est ajoutée dans la composition préalablement chauffée contenant les tensioactifs et les composés hydrosolubles.

L'invention a également pour objet un procédé de lavage et de conditionnement des matières kératiniques telles que notamment les cheveux consistant à appliquer sur lesdites matières mouillées une quantité efficace d'une composition telle que définie ci-dessus, puis à effectuer un rinçage à l'eau après un éventuel temps de pause.

Les compositions selon l'invention sont utilisées de préférence comme shampooings pour le lavage et le conditionnement des cheveux et ils sont appliqués dans ce cas-là sur les cheveux humides dans des quantités efficaces pour les laver, et la mousse générée par massage ou friction avec les mains est ensuite éliminée après un éventuel temps de pause, par rinçage à l'eau, l'opération pouvant être répétée une ou plusieurs fois.

Les compositions conformes à l'invention sont également utilisables comme gels douche pour le lavage et le conditionnement des cheveux et/ou de la peau, auquel cas ils sont appliqués sur la peau et/ou les cheveux humides et sont rincés après application.

Des exemples concrets, mais nullement limitatifs, illustrant l'invention vont maintenant être donnés.

### EXEMPLE 1

### Influence du rapport TA amphotère/TA anionique

On a réalisé deux compositions de shampooings, l'une conforme à l'invention (composition A) et l'autre comparative (composition B) :

| | B Comparatif | A Invention |
|---|---|---|
| - Lauryléthersulfate de sodium (C12/C14 à 70/30) à 2,2 moles d'oxyde d'éthylène en solution aqueuse à 70% de MA (MA = matière active) | 16,8 gMA | 15 gMA |
| - Cocoylbétaïne (DEHYTON AB 30) | 2,4 gMA | 5 gMA |
| - Triméthylsilyl amodiméthicone indice d'amine 0,6 meq./g (FINISH WT 1650 de WACKER) | 3 g | 3 g |
| - Homopolymère de chlorure de diallyl diméthyl ammonium en solution aqueuse à 40% de MA (MERQUAT 100 de CALGON) | 0,4 gMA | 0,4 gMA |
| - NaCl | 3,25 g | 3,25 g |
| - Parfum, conservateur | qs | qs |
| - Acide chlorhydrique qs pH | 6 | 6 |
| - Eau déminéralisée qs | 100 g | 100 g |

La composition A selon l'invention (TA amphotère/TA anionique = 0,33) est transparente.

La composition B comparative (TA amphotère/TA anionique = 0,14) n'est pas transparente.

La transparence est évaluée par turbidimétrie avec une valeur inférieure à 25 NTU (Nephelometric turbidity units).

On a effectué un shampooing en appliquant environ 1 g de la composition A sur des mèches de cheveux sensibilisés (2,5 g) préalablement mouillés. On fait mousser le shampooing, on laisse poser 10 mn puis on rince abondamment à l'eau. On essore les mèches.
On procède selon le même mode opératoire que ci-dessus avec la composition comparative B.

Un panel d'experts a évalué l'aspect des cheveux.

Tous les experts indiquent que les cheveux traités avec la composition A selon l'invention se démêlent plus facilement, sont plus doux et plus lisses que les cheveux traités avec la composition B.

### EXEMPLE 2

### Influence de l'indice d'amine

On a réalisé des compositions de shampooings de composition suivante :

| | A |
|---|---|
| - Lauryléthersulfate de sodium (C12/C14 à 70/30) à 2,2 moles d'oxyde d'éthylène en solution aqueuse à 70% de MA (MA = matière active) | 15 gMA |
| - Cocoylbétaïne (DEHYTON AB 30) | 5 gMA |
| - Silicone aminée | 3 g |
| - Homopolymère de chlorure de diallyl diméthyl ammonium en solution aqueuse à 40% de MA (MERQUAT 100 de CALGON) | 0,4 gMA |
| - NaCI | 3,25 g |
| - Parfum, conservateur | qs |
| - Acide chlorhydrique qs pH | 6 |
| - Eau déminéralisée qs | 100 g |

Les silicones testées ont été les suivantes et les résultats sont rassemblés dans le tableau suivant :

| | SILICONE AMINEE | | Indice d'amine (meq./g) | Transparence |
|---|---|---|---|---|
| **1** | Triméthylsilyl amodiméthicone | VP 1480 M (WACKER) | 0,12-0,15 | NON |
| **2** | Triméthylsilyl amodiméthicone | Silicone Fluid F801 (WACKER) | 0,14 | NON |
| **3** | Amodimethicone | FINISH WR 100 (WACKER) | 0,15 | NON |
| **4** | Amodimethicone | FINISH WR 1300 (WACKER) | 0,3 | NON |
| **5** | Amodimethicone en émulsion | SILSOFT TP 515 (OSI) | 0,058 | NON |
| **6** | Amodimethicone en émulsion | DC939 (DOW CORNING) | <0,1 | NON |
| **7** | Triméthylsilyl amodimethicone | FINISH WT 1650 (WACKER) | 0,6 | OUI |
| **8** | Triméthylsilyl amodimethicone | SF 1708 (General Electric) | 0,8 | OUI |
| **9** | Amodimethicone | FINISH WT 1600 (WACKER) | 0,6 | OUI |
| **10** | Amodimethicone | SP4 Silicone Fluid (GENESE) | 0,75 | OUI |
| **11** | Amodimethicone | L650 (WACKER) | 2,7-3,2 | OUI |

Les compositions 7 à 11 selon l'invention sont transparentes alors que les compositions (1 à 6) contenant une silicone ayant un indice d'amine inférieur à 0,4 meq./g ne sont pas transparentes.
Les compositions 1 à 6 sont stables alors que les compositions 7 à 11 ne le sont pas (la silicone relargue à la surface de la composition).

## Revendications

1. Composition cosmétique détergente et conditionnante, **caractérisée par le fait qu'**elle comprend, dans un milieu aqueux cosmétiquement acceptable, (A) une base lavante comprenant au moins un tensioactif anionique et au moins un tensioactif amphotère, (B) un système conditionneur comprenant au moins une silicone aminée dont l'indice d'amine est supérieur ou égal à 0,4 meq./g, choisie parmi:
(a) les polymères cationiques siliconés répondant à la formule :
R'ₐG₃₋ₐ-Si(OSiG₂)ₙ-(OSiG_{b}R'_{2-b})ₘ-O-SiG₃₋ₐ-R'ₐ (I)
dans laquelle :
G est un atome d'hydrogène, ou un groupement phényle, OH, ou alkyle en C₁-C₈, par exemple méthyle,
a désigne le nombre 0 ou un nombre entier de 1 à 3, en particulier 0,
b désigne 0 ou 1, et en particulier 1,
m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
R' est un radical monovalent de formule -C_{q}H_{2q}L dans laquelle q est un nombre de 2 à 8 et L est un groupement aminé éventuellement quaternisé choisi parmi les groupements :
-N(R")-CH₂-CH₂-N(R")₂
-N(R")₂
-N^{⊕}(R")₃A⁻
-N^{⊕}H(R")₂A⁻
-N^{⊕}H₂(R")A⁻
-N(R")-CH₂-CH₂-N^{⊕}R"H₂A⁻,
dans lesquels R", identiques ou différents, peut désigner hydrogène, phényle, benzyle, ou un radical hydrocarboné saturé monovalent, par exemple un radical alkyle ayant de 1 à 20 atomes de carbone, de préférence méthyle et A⁻ représente un anion organique ou minéral.
(b) les polymères cationiques siliconés répondant à la formule (IV) suivante : 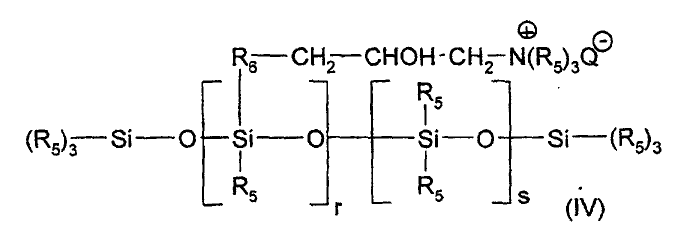 dans laquelle
R₅ représente un radical hydrocarboné monovalent ayant de 1 à 18 atomes de carbone, et en particulier un radical alkyle en C₁-C₁₈, ou alcényle en C₂-C₁₈, par exemple méthyle ;
R₆ représente un radical hydrocarboné divalent, notamment un radical alkylène en C₁-C₁₈ ou un radical alkylèneoxy divalent en C₁-C₁₈, par exemple en C₁-C₈ ;
Q⁻ est un anion organique ou minéral ;
r représente une valeur statistique moyenne de 2 à 20 et en particulier de 2 à 8 ;
s représente une valeur statistique moyenne de 20 à 200 et en particulier de 20 à 50,
le rapport en poids tensioactif amphotère / tensioactif anionique étant supérieur ou égal à 0,2.

2. Composition selon la revendication 1, **caractérisée par le fait que** la silicone aminée a un indice d'amine compris entre 0,5 et 5 meq./g.

3. Composition selon l'une quelconque des revendications 1 ou 2, **caractérisée par le fait que** ladite base lavante est présente à une teneur pondérale comprise entre 4 % et 50 % en poids par rapport au poids total de la composition, de préférence comprise entre 6 % et 35 % en poids et plus préférentiellement comprise entre 8 % et 25 % en poids.

4. Composition selon l'une quelconque des revendications 1 à 3, **caractérisée en ce que** le(s) tensioactif(s) anionique(s) est (sont) présent(s) dans des concentrations allant de 3 à 30 % en poids, de préférence de 5 à 20 % en poids, par rapport au poids total de la composition.

5. Composition selon l'une quelconque des revendications 1 à 4, **caractérisée en ce que** le(s) tensioactif(s) amphotère(s) est(sont) présent(s) dans des concentrations allant de 1 à 20 % en poids, de préférence de 1,5 à 15 % en poids, par rapport au poids total de la composition.

6. Composition selon l'une quelconque des revendications 1 à 5, caractérisée le rapport en poids tensioactif amphotère / tensioactif anionique est compris entre 0,2 et 10, plus particulièrement entre 0,25 et 5 et encore plus particulièrement entre 0,3 et 3.

7. Composition selon l'une quelconque des revendications 1 à 6, **caractérisée en ce que** la silicone aminée est choisie parmi :
- le polymère dénommé "triméthylsilylamodiméthicone", répondant à la formule 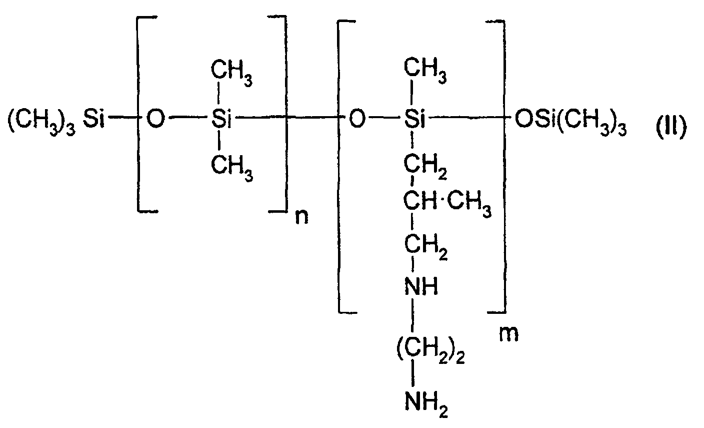 dans laquelle m et n sont des nombres tels que la somme (n + m) peut varier notamment de 1 à 2 000, n pouvant désigner un nombre de 0 à 1 999 et notamment de 49 à 149 et m pouvant désigner un nombre de 1 à 2 000, et notamment de 1 à 10 ;
- le polymère dénommé "amodiméthicone" répondant à la formule : 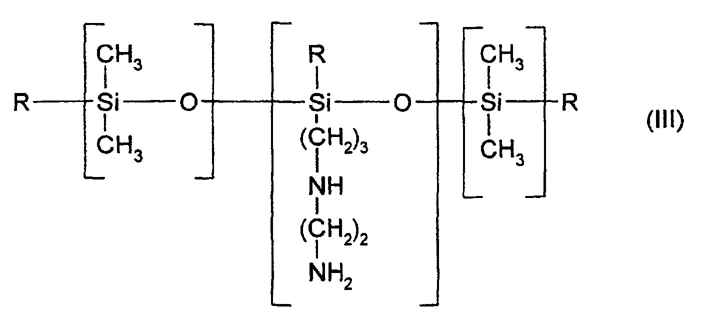
dans laquelle R désigne OH ou méthyle.

8. Composition selon l'une quelconque des revendications 1 à 7, **caractérisée par le fait que** la silicone est présente dans des concentrations comprises entre 0,05 et 15 %, et de préférence entre 0,2 et 10 % en poids par rapport au poids total de la composition.

9. Composition selon l'une quelconque des revendications 1 à 8, **caractérisée par le fait qu'**elle contient en plus un ou plusieurs adjuvants choisis par les agents tensioactifs cationiques, les polymères anioniques ou non ioniques ou cationiques ou amphotères, les protéines, les hydrolysats de protéines, les céramides, les pseudocéramides, les acides gras à chaînes linéaires ou ramifiées en C₁₆-C₄₀ tels que l'acide méthyl-18 eicosanoïque, les hydroxyacides, les vitamines, le panthénol, les silicones différentes des silicones de l'invention, volatiles ou non volatiles, solubles et insolubles dans le milieu, les filtres UV, les agents hydratants, les agents antipelliculaires ou antiséborrhéiques, les agents anti-radicaux libres, et leurs mélanges.

10. Composition selon la revendication 9, **caractérisée par** le fait le polymère cationique est choisi parmi les dérivés d'éther de cellulose quaternaires, les homopolymères de sel de diallyldiméthylammonium et les copolymères de sel de diallyldiméthylammonium et d'acrylamide, les polysaccharides cationiques, les copolymères de vinylpyrrolidone et de sel de méthylvinyl imidazolium.

11. Composition selon la revendication 9, **caractérisée par** le fait le polymère cationique est choisi parmi les polymères qui sont constitués de motifs récurrents répondant à la formule : 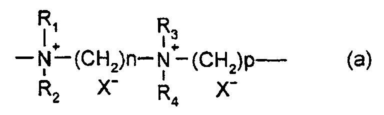 dans laquelle R₁, R₂, R₃ et R_{4,} identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.

12. Composition selon l'une quelconque des revendications 9 à 11, **caractérisée en ce que** le polymère cationique représente de 0,001 % à 10 % en poids, de préférence de 0,005 % à 5 % en poids, et encore plus préférentiellement de 0,01 % à 3 % en poids, du poids total de la composition.

13. Composition selon l'une quelconque des revendications 1 à 12, **caractérisée par** le fait le milieu aqueux cosmétiquement acceptable est constitué uniquement par de l'eau ou par un mélange d'eau et d'un solvant cosmétiquement acceptable tel qu'un alcool inférieur en C₁-C₁₂, comme l'éthanol, l'isopropanol, le tertiobutanol, le n-butanol, l'hexanol, le décanol ; les polyols tels que les alkylèneglycols comme le propylèneglycol, la glycérine et les polyalkylèneglycols ; les éthers de glycols.

14. Composition selon la revendication 13, **caractérisée en ce que** le solvant est présent dans des concentrations comprises entre 0,1 et 20% en poids par rapport au poids total de la composition.

15. Utilisation d'une composition telle définie dans l'une quelconque des revendications 1 à 14 pour le nettoyage et/ou le démaquillage et/ou le conditionnement des matières kératiniques.

16. Procédé de lavage et de conditionnement des matières kératiniques telles que les cheveux consistant à appliquer sur lesdites matières mouillées une quantité efficace d'une composition telle que définie dans l'une quelconque des revendications 1 à 14, puis à effectuer un rinçage à l'eau après un éventuel temps de pause.

## Patentansprüche

1. Reinigende und konditionierende kosmetische Zusammensetzung, **dadurch gekennzeichnet, dass** sie in einem kosmetisch akzeptablen, wässrigen Medium (A) eine reinigende Basisformulierung, die mindestens einen anionischen grenzflächenaktiven Stoff und mindestens einen amphoteren grenzflächenaktiven Stoff enthält, und (B) ein Konditioniersystem enthält, das mindestens ein aminiertes Silicon mit einem Aminoindex von 0,4 meq/g oder darüber enthält, das ausgewählt ist unter:
a) den kationischen siliconierten Polymeren der folgenden Formel:
R'ₐG₃₋ₐ-Si(OSiG₂)ₙ-(OSiG_{b}R'_{2-b})ₘ-O-SiG₃₋ₐ-R'ₐ (I),
worin bedeuten:
· die Gruppe G Wasserstoff, Phenyl, OH oder C₁₋₈-Alkyl, beispielsweise Methyl,
· a 0 oder eine ganze Zahl von 1 bis 3 und insbesondere 0,
· b 0 oder 1 und insbesondere 1,
· m und n Zahlen, die so ausgewählt sind, dass die Summe (n + m) insbesondere im Bereich von 1 bis 2000 und besonders 50 bis 150 liegen kann, wobei n eine Zahl von 0 bis 1999 und insbesondere 49 bis 149 und m eine Zahl von 1 bis 2000 und insbesondere 1 bis 10 bedeuten kann,
· die Gruppe R' eine einwertige Gruppe der Formel -C_{q}H_{2q}L, worin q eine Zahl von 2 bis 8 ist und L eine gegebenenfalls quaternisierte aminierte Gruppe bedeutet, welche unter den folgenden Gruppen ausgewählt ist:
- NR"-CH₂-CH₂-N(R")₂
- N(R")₂
- N^{⊕}(R")₃A⁻
- N^{⊕}H(R")₂A⁻
- N^{⊕}H₂(R")A⁻
- N(R")-CH₂-CH₂-N^{⊕}R"H₂A⁻,
worin die Gruppen R", die gleich oder verschieden sind, Wasserstoff, Phenyl, Benzyl oder eine einwertige gesättigte Kohlenwasserstoffgruppe, beispielsweise eine Alkylgruppe mit 1 bis 20 Kohlenstoffatomen und vorzugsweise Methyl bedeuten können und A- ein organisches oder anorganisches Anion bedeutet;
b) den kationischen siliconierten Polymeren der folgenden Formel (IV):
R₆-CH₂-CHOH-CH₂-N⁺(R₅)₃ Q⁻ 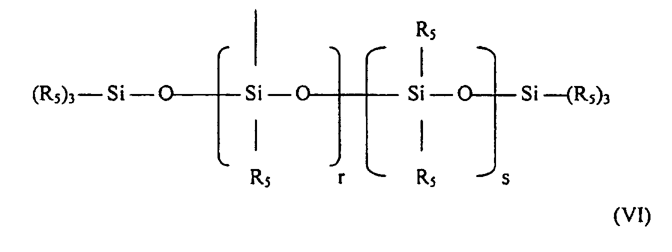
worin bedeuten:
- R₅ eine einwertige Kohlenwasserstoffgruppe mit 1 bis 18 Kohlenstoffatomen und insbesondere eine C₁₋₁₈-Alkylgruppe oder eine C₂₋₁₈-Alkenylgruppe, beispielsweise Methyl,
- R₆ eine zweiwertige Kohlenwasserstoffgruppe und insbesondere eine Alkylengruppe mit 1 bis 18 Kohlenstoffatomen oder eine zweiwertige Alkylenoxygruppe mit 1 bis 18 Kohlenstoffatomen, beispielsweise mit 1 bis 8 Kohlenstoffatomen,
- Q⁻ ein organisches oder anorganisches Anion,
- r einen statistischen Mittelwert von 2 bis 20 und insbesondere von 2 bis 8, und
- s einen statistischen Mittelwert von 20 bis 200 und insbesondere von 20 bis 50,
wobei das Gewichtsverhältnis amphoterer grenzflächenaktiver Stoff/anionischer grenzflächenaktiver Stoff 0,2 beträgt oder darüber liegt.

2. Zusammensetzung nach Anspruch 1, **dadurch gekennzeichnet, dass** das aminierte Silicon einen Aminoindex von 0,5 bis 5 meq/g aufweist.

3. Zusammensetzung nach einem der Ansprüche 1 oder 2, **dadurch gekennzeichnet, dass** die reinigende Basisformulierung in einem Gewichtsanteil von 4 bis 50 %, bezogen auf das Gesamtgewicht der Zusammensetzung, vorzugsweise 6 bis 35 Gew.-% und insbesondere 8 bis 25 Gew.-% vorliegt.

4. Zusammensetzung nach einem der Ansprüche 1 bis 3, **dadurch gekennzeichnet, dass** der (oder die) anionische(n) grenzflächenaktive(n) Stoff(e) in Konzentrationen von 3 bis 30 Gew.-% und vorzugsweise 5 bis 20 Gew.-%, bezogen auf das Gesamtgewicht der Zusammensetzung, vorliegt (vorliegen).

5. Zusammensetzung nach einem der Ansprüche 1 bis 4, **dadurch gekennzeichnet, dass** der (oder die) amphotere(n) grenzflächenaktive(n) Stoff(e) in Konzentrationen von 1 bis 20 Gew.-% und vorzugsweise 1,5 bis 15 Gew.-%, bezogen auf das Gesamtgewicht der Zusammensetzung, vorliegt (vorliegen).

6. Zusammensetzung nach einem der Ansprüche 1 bis 5, **dadurch gekennzeichnet, dass** das Gewichtsverhältnis amphoterer grenzflächenaktiver Stoff / anionischer grenzflächenaktiver Stoff im Bereich von 0,2 bis 10, insbesondere 0,25 bis 5 und besonders 0,3 bis 3 liegt.

7. Zusammensetzung nach einem der Ansprüche 1 bis 6, **dadurch gekennzeichnet, dass** das aminierte Silicon ausgewählt ist unter:
- dem als Trimethylsilylamodimeticon bezeichneten Polymer der folgenden Formel: 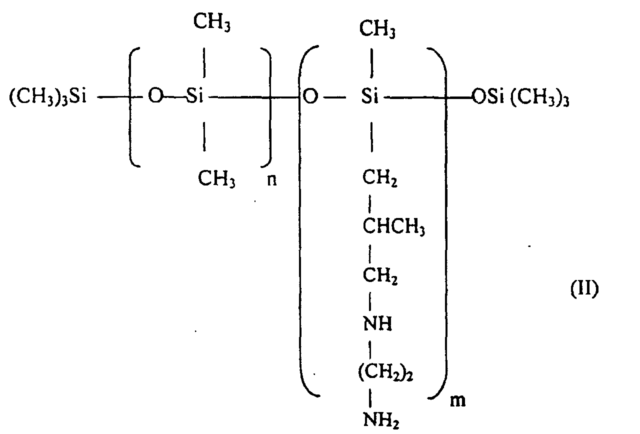 worin n und m Zahlen bedeuten, die so ausgewählt sind, dass die Summe (n+m) insbesondere im Bereich von 1 bis 2000 liegt, wobei n eine Zahl von 0 bis 1999 und insbesondere 49 bis 149 und m eine Zahl von 1 bis 2000 und insbesondere 1 bis 10 bedeuten kann;
- dem als "Amodimeticon" bezeichneten Polymer der folgenden Formel: 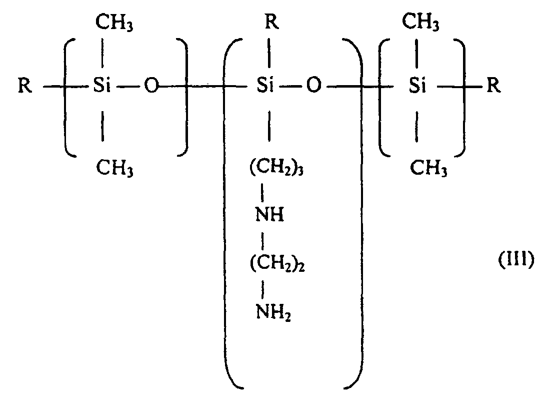
worin R OH oder Methyl bedeutet.

8. Zusammensetzung nach einem der Ansprüche 1 bis 7, **dadurch gekennzeichnet, dass** das Silicon in Konzentrationen von 0,05 bis 15 Gew.-% und vorzugsweise 0,2 bis 10 Gew.-%, bezogen auf das Gesamtgewicht der Zusammensetzung, vorliegt.

9. Zusammensetzung nach einem der Ansprüche 1 bis 8, **dadurch gekennzeichnet, dass** sie ferner einen oder mehrere Zusatzstoff enthält die unter kationischen grenzflächenaktiven Stoffen, anionischen, nichtionischen, kationischen oder amphoteren Polymeren, Proteinen, Proteinhydrolysaten, Ceramiden, Pseudoceramiden, Fettsäuren mit linearen oder verzweigten C₁₆₋₄₀-Fettketten, wie 18-Methyleicosansäure, Hydroxysäuren, Vitaminen, Panthenol, flüchtigen oder nicht flüchtigen Siliconen, die in dem Medium löslich oder unlöslich und von den erfindungsgemäßen Siliconen verschieden sind, UV-Filtern, Hydratisierungsmitteln, Wirkstoffen gegen Haarausfall oder gegen Seborrhoe, Radikalfängern für freie Radikale und deren Gemischen ausgewählt sind.

10. Zusammensetzung nach Anspruch 9, **dadurch gekennzeichnet, dass** das kationische Polymer unter quartären Celluloseetherderivaten, Homopolymeren von Diallyldimethylammoniumsalzen und Copolymeren von Diallyldimethylammoniumsalzen und Acrylamid, kationischen Polysacchariden und Copolymeren von Vinylpyrrolidon und Methylvinylimidazoliumsalzen ausgewählt ist.

11. Zusammensetzung nach Anspruch 9, **dadurch gekennzeichnet, dass** das kationische Polymer unter den Polymeren ausgewählt ist, die aus wiederkehrenden Einheiten der folgenden Formel bestehen: 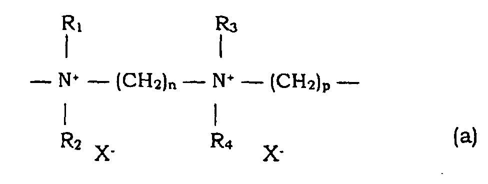 wobei die Gruppen R₁, R₂, R₃ und R₄, die identisch oder voneinander verschieden sind, eine Alkyl- oder Hydroxyalkylgruppe mit etwa 1 bis 4 Kohlenstoffatomen bedeuten, n und p ganze Zahlen im Bereich von etwa 2 bis 20 sind und X⁻ ein von einer anorganischen oder organischen Säure abgeleitetes Anion ist.

12. Zusammensetzung nach einem der Ansprüche 9 bis 11, **dadurch gekennzeichnet, dass** das kanonische Polymer 0,001 bis 10 Gew.-%, vorzugsweise 0,005 bis 5 Gew.-% und noch bevorzugter 0,01 bis 3 Gew.-%, bezogen auf das Gesamtgewicht der Zusammensetzung, ausmacht.

13. Zusammensetzung nach einem der Ansprüche 1 bis 12, **dadurch gekennzeichnet, dass** das kosmetisch akzeptable, wässrige Medium ausschließlich aus Wasser oder einem Gemisch von Wasser und einem kosmetisch akzeptablen Lösungsmittel besteht, beispielsweise einem niederen C₁₋₁₂-Mkohol, wie Ethanol, Isopropanol, *t*-Butanol, n-Butanol, Hexanol oder Decanol; Polyolen, wie Alkylenglykolen, beispielsweise Propylenglykol, Glycerin oder Polyalkylenglykolen; und Glykolethern.

14. Zusammensetzung nach Anspruch 13, **dadurch gekennzeichnet, dass** das Lösungsmittel in Konzentrationen von 0,1 bis 20 Gew.-%, bezogen auf das Gesamtgewicht der Zusammensetzung, vorliegt.

15. Verwendung einer Zusammensetzung nach einem der Ansprüche 1 bis 14 zur Reinigung und/oder zum Abschminken und/oder zum Konditionieren von Keratinsubstanzen.

16. Verfahren zum Waschen und zum Konditionieren von Keratinsubstanzen, wie dem Haar, das darin besteht, auf die feuchten Keratinsubstanzen eine wirksame Menge einer Zusammensetzung nach einem der Ansprüche 1 bis 14 aufzutragen und anschließend, gegebenenfalls nach einer Einwirkzeit, mit Wasser zu spülen

## Claims

1. Detergent and conditioning cosmetic composition, **characterized in that** it comprises, in a cosmetically acceptable aqueous medium, (A) a washing base comprising at least one anionic surfactant and at least one amphoteric surfactant and (B) a conditioner system comprising at least one aminated silicone, the amine number of which is greater than or equal to 0.4 meq/g, chosen from :
(a) cationic silicone polymers corresponding to the formula:
R'ₐG₃₋ₐ-Si(OSiG₂)ₙ-(OSiG_{b}R'_{2-b})ₘ-O-SiG₃₋ₐ-R'ₐ (I)
in which:
G is a hydrogen atom or a phenyl, OH or C₁-C₈ alkyl group, for example a methyl group,
a denotes the number 0 or an integer from 1 to 3, in particular 0,
b denotes 0 or 1 and in particular 1,
m and n are numbers such that the sum (n+m) can vary in particular from 1 to 2000 and in particular from 50 to 150, it being possible for n to denote a number from 0 to 1999 and in particular from 49 to 149 and it being possible for m to denote a number from 1 to 2000 and in particular from 1 to 10;
R' is a monovalent radical of formula -C_{q}H_{2q}L, in which q is a number from 2 to 8 and L is an optionally quaternized amino group chosen from the groups:
- N(R")-CH₂-CH₂-N(R")₂
- N(R")₂
- N^{⊕}(R")₃A⁻
- N^{⊕}H(R")₂A⁻
- N^{⊕}H₂(R")A⁻
- N(R") -CH₂-CH₂-N^{⊕}R"H₂A⁻,
in which R", which are identical or different, can denote hydrogen, phenyl, benzyl or a saturated monovalent hydrocarbon-comprising radical, for example an alkyl radical having from 1 to 20 carbon atoms, preferably a methyl radical, and A⁻ represents an organic or inorganic anion,
(b) cationic silicone polymers corresponding to the following formula (IV): 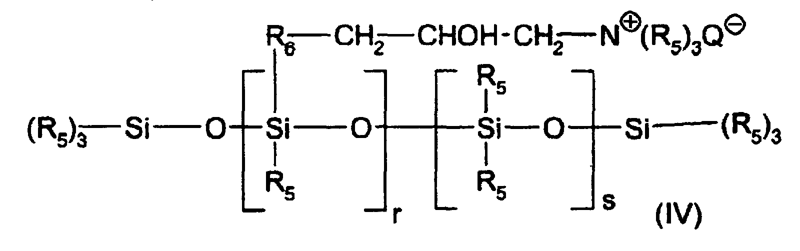 in which:
R₅ represents a monovalent hydrocarbon-comprising radical having from 1 to 18 carbon atoms and in particular a C₁-C₁₈ alkyl or C₂-C₁₈ alkenyl radical, for example a methyl radical;
R₆ represents a divalent hydrocarbon-comprising radical, in particular a C₁-C₁₈ alkylene radical or a divalent C₁-C₁₈ alkyleneoxy radical, for example a C₁-C₈ radical;
Q⁻ is an organic or inorganic anion;
r represents a mean statistical value of 2 to 20 and in particular of 2 to 8;
s represents a mean statistical value of 20 to 200 and in particular of 20 to 50,
the amphoteric surfactant/anionic surfactant ratio by weight being greater than or equal to 0.2.

2. Composition according to Claim 1, **characterized in that** the aminated silicone has an amine number of between 0.5 and 5 meq/g.

3. Composition according to either one of Claims 1 and 2, **characterized in that** the said washing base is present at a content by weight of between 4% and 50% by weight with respect to the total weight of the composition, preferably of between 6% and 35% by weight and more preferably of between 8% and 25% by weight.

4. Composition according to any one of Claims 1 to 3, **characterized in that** the anionic surfactant(s) is (are) present in concentrations ranging from 3 to 30% by weight, preferably from 5 to 20% by weight, with respect to the total weight of the composition.

5. Composition according to any one of Claims 1 to 4, **characterized in that** the amphoteric surfactant(s) is (are) present in concentrations ranging from 1 to 20% by weight, preferably from 1.5 to 15% by weight, with respect to the total weight of the composition.

6. Composition according to any one of Claims 1 to 5, **characterized in that** the amphoteric surfactant/anionic surfactant ratio by weight is between 0.2 and 10, more particularly between 0.25 and 5 and more particularly still between 0.3 and 3.

7. Composition according to any one of Claims 1 to 6, **characterized in that** the aminated silicone is chosen from:
- the polymer named "trimethylsilylamodimethicone", corresponding to the formula: 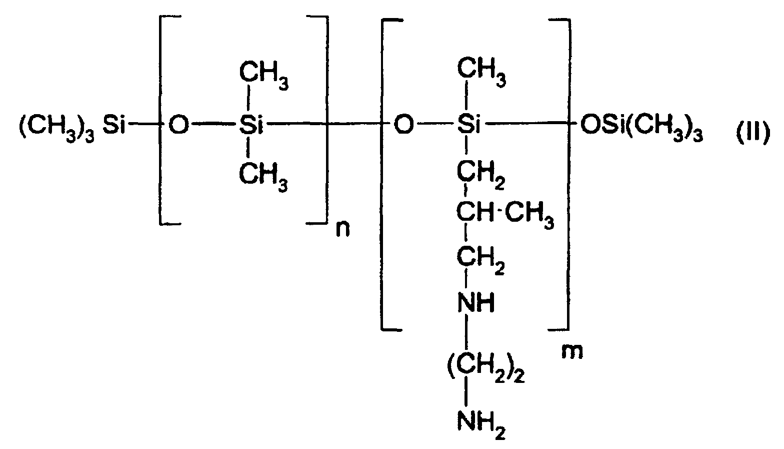 in which m and n are numbers such that the sum (n+m) can vary in particular from 1 to 2000, it being possible for n to denote a number from 0 to 1999 and in particular from 49 to 149 and it being possible for m to denote a number from 1 to 2000 and in particular from 1 to 10;
- the polymer named "amodimethicone", corresponding to the formula: 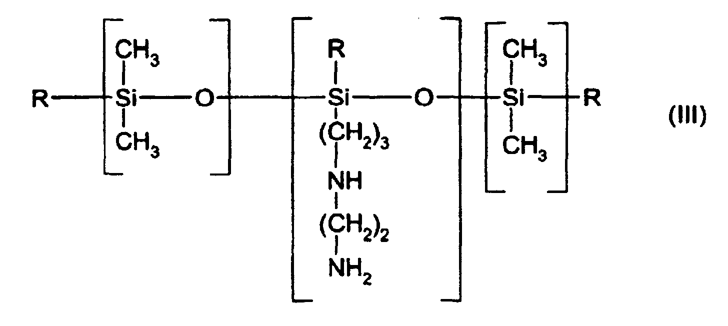
in which R denotes OH or methyl.

8. Composition according to any one of Claims 1 to 7, **characterized in that** the silicone is present in concentrations of between 0.05 and 15% and preferably between 0.2 and 10% by weight with respect to the total weight of the composition.

9. Composition according to any one of Claims 1 to 8, **characterized in that** it additionally comprises one or more adjuvants chosen from cationic surface-active agents, anionic or non-ionic or cationic or amphoteric polymers, proteins, protein hydrolysates, ceramides, pseudoceramides, fatty acids comprising linear or branched C₁₆-C₄₀ chains, such as 18-methyl-eicosanoic acid, hydroxy acids, vitamins, panthenol, volatile or non-volatile silicones, other than the silicones of the invention, which are soluble or insoluble in the medium, UV screening agents, moisturizing agents, antidandruff or antiseborrhoeic agents, agents for combating free radicals, and their mixtures.

10. Composition according to Claim 9, **characterized in that** the cationic polymer is chosen from quaternary derivatives of cellulose ether, diallyldimethylammonium salt homopolymers and copolymers of diallyldimethylammonium salt and of acrylamide, cationic polysaccharides, or copolymers of vinylpyrrolidone and of methylvinylimidazolium salt.

11. Composition according to Claim 9, **characterized in that** the cationic polymer is chosen from polymers which are composed of repeat units corresponding to the formula: 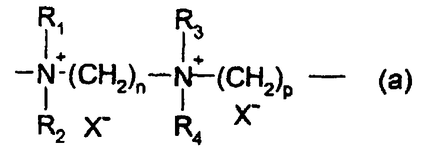 in which R₁, R₂, R₃ and R₄, which are identical or different, denote an alkyl or hydroxyalkyl radical having from 1 to 4 carbon atoms approximately, n and p are integers varying from 2 to 20 approximately and X⁻ is an anion derived from an inorganic or organic acid.

12. Composition according to any one of Claims 9 to 11, **characterized in that** the cationic polymer represents from 0.001% to 10% by weight, preferably from 0.005% to 5% by weight and more preferably still from 0.01% to 3% by weight of the total weight of the composition.

13. Composition according to any one of Claims 1 to 12, **characterized in that** the cosmetically acceptable aqueous medium is composed solely of water or of a mixture of water and of a cosmetically acceptable solvent, such as a lower C₁-C₁₂ alcohol, for example ethanol, isopropanol, tert-butanol, n-butanol, hexanol or decanol; polyols, such as alkylene glycols, for example propylene glycol, glycerol and poly(alkylene glycol)s; or glycol ethers.

14. Composition according to Claim 13, **characterized in that** the solvent is present in concentrations of between 0.1 and 20% by weight with respect to the total weight of the composition.

15. Use of a composition as defined in any one of Claims 1 to 14 for cleaning and/or removing make-up from and/or conditioning keratinous substances.

16. Process for washing and for conditioning keratinous substances, such as the hair, which consists in applying, to the wetted said substances, an effective amount of a composition as defined in any one of Claims 1 to 14 and in then rinsing with water, after an optional period of rest.
